# EUROPÄISCHE PATENTSCHRIFT

(11) **EP 1 010 436 B1**
(45) Veröffentlichungstag und Bekanntmachung des Hinweises auf die Patenterteilung: **16.10.2002**
(21) Anmeldenummer: 99124374.2
(22) Anmeldetag: 07.12.1999
(51) Int. Cl.: A61L 24/04, A61L 27/18

(54) **Knochensiegel**
Bone wax composition
Cire osseuse

(30) Priorität: 19.12.1998 DE 19858891
(43) Veröffentlichungstag der Anmeldung: 21.06.2000
(73) Patentinhaber: MERCK PATENT GmbH, 64293 Darmstadt (DE)
(72) Erfinder: Ritter, Wolfgang, Dr., 67591 Offstein (DE); Wenz, Robert, Dr., 61206 Wöllstadt (DE); Krotz, Ralf, Dr., 69168 Wiesloch (DE)

(56) Entgegenhaltungen:
- DE-A- 3 716 302
- DE-A- 4 235 312
- GB-A- 1 584 080

## Beschreibung

Die vorliegende Erfindung betrifft verbesserte Knochensiegel zum Stillen lokaler Blutungen am Knochen.

Knochensiegel oder Knochenwachse dienen zum mechanischen Verschluss blutender Knochenwunden. Resorbierbare Knochensiegel haben den Vorteil gegenüber den nicht resorbierbaren Knochenwachsen, daß sie vom Körper vollständig verstoffwechselt werden, ohne entzündliche Erscheinungen, insbesondere makrophagen-induzierte Fremdkörperreaktionen, hervorzurufen.

Solche resorbierbaren Wachse zur mechanischen Blutstillung an körpereigenem Hartgewebe, insbesondere Knochen, sind bekannt und beispielsweise Gegenstand der EP 0 100 981. Die dort beschriebenen Wachse oder Siegel zeichnen sich dadurch aus, daß sie aus bei Körpertemperatur zähviskosen bis festen wachsartigen Polyesteroligomeren von niederen Hydroxycarbonsäuren bestehen. Aufgrund ihrer Struktur sind diese Wachse durch körpereigene Stoffwechselmechanismen abbaubar, wobei die Geschwindigkeit des Abbaus in an sich bekannter Weise einstellbar ist. Oligomere der Glykolsäure werden vom körpereigenen Stoffwechsel schneller abgebaut als solche der Milchsäure. Die Abbaugeschwindigkeit ist damit beispielweise durch Mischveresterung der beiden genannten Oxycarbonsäuren regulierbar. Die bevorzugten Wachse weisen mittlere Molekulargewichte im Bereich von etwa 200 bis 1 500 und insbesondere im Bereich von etwas 300 bis 1 000 auf.

Zur Regelung des mittleren Molekulargewichts dieser Polyesteroligomeren schlägt die genannte Patentschrift vor, monofunktionelle und/oder difunktionelle Alkohole oder Carbonsäuren bzw. Carbonsäure-Anhydride und/oder primäre bzw. sekundäre Monoamine mitzuverwenden. In an sich bekannter Weise kann dann durch Wahl geeigneter Mischungsverhältnisse an Oxycarbonsäuren und zusätzlicher monofunktioneller bzw. difunktioneller Komponente ein sich letztlich einstellendes mittleres Molekulargewicht vorherbestimmt werden.

Gegenstand der GB 15 84 080 (Ethicon) ist die Verwendung von wasserlöslichen Polymeren, insbesondere Polyethylenglykol mit Molmassen von 200-4000, in Knochensiegelmassen.

Gegenstand der DE-PS 37 16 302 ist eine weiterführende Optimierung solcher resorbierbarer Wachse. Beschrieben ist in diesem Schutzrecht, daß besonders körper- und gewebeverträgliche Wachse dann erhalten werden, wenn - unter Wahrung der allgemeinen Gesetzmäßigkeiten aus der vorher genannten Schrift - zur Einstellung des mittleren Molekulargewichts ein ganz bestimmter dreifunktioneller Alkohol, nämlich Glycerin, eingesetzt wird. Die Kombination von Glycerin mit Oligoestern der Milchsäure und/oder der Glykolsäure führt zu abbaubaren wachsartigen Komponenten der genannten Art, die sich bei der Implantation in lebendes Körpergewebe durch eine besonders gut ausgeprägte Körperverträglichkeit auszeichnen.

Bei der praktischen Anwendung dieser bekannten und gut verträglichen Substanzen zeigen sich jedoch auch weiterhin Nachteile wie z.B. eine starke Adhäsion an Kunststoffe, schlechte Extrudierbarkeit oder auch eine nicht optimale Viskosität bei Raumtemperatur.

Die Lehre der vorliegenden Erfindung geht daher von der Aufgabe aus, körperresorbierbare Knochensiegel in mehrfacher Weise weiterhin zu verbessern. Insbesondere sollen Knochensiegelmassen zur Verfügung gestellt werden, die nicht die Nachteile einer starken Adhäsion an Kunststoffen, wie z.B. chirurgischen Handschuhen, haben und ferner eine verbesserte Extrudierbarkeit aus Verpackungsmaterialien bei verbesserten mechanischen Eigenschaften, insbesondere der Viskosität bei Raumtemperatur und bei Körpertemperatur, aufweisen unter Beibehaltung der blutstillenden Eigenschaften.

Die Lehre der Erfindung zur Lösung dieser vielseitigen Aufgabenstellung geht von der Erkenntnis aus, daß ein solch anspruchsvoller Knochensiegel nur in einer ganz bestimmten Abstimmung der Einzelkomponenten erhalten werden kann. Überraschenderweise wurde nun gefunden, daß die Abmischung von Polyesteroligomeren ganz bestimmter Zusammensetzungen mit dem wasserlöslichen Polymer Polyethylenglykol (PEG) zu den in vieler Hinsicht verbesserten Knochensiegelmassen führen.

Gegenstand der Erfindung ist dementsprechend ein Knochensiegel zur Stillung lokaler Blutungen am Knochen, dadurch gekennzeichnet, daß es aus einem Polyesteroligomeren, aufgebaut aus Glycerin Lactid, Glykolid und dem wasserlöslichen Polymer PEG besteht.

Die Oligomersegmente der erfindungsgemäß beschriebenen optimierten Knochenwachse leiten sich von der Milchsäure und Glykolsäure ab. Im Allgemeinen setzt man die leicht handhabbaren Dimerisationsprodukte, d.h. das Lactid und das Glykolid, ein, jedoch ist es auch möglich, die monomeren Hydroxycarbonsäuren als Ausgangsmaterial zu nehmen. Die Milchsäure bzw. das Milchsäuredimere kann als optisch aktive Komponente oder auch als ein Gemisch der optisch aktiven Verbindungen als D,L-Lactid Verwendung finden.

Als dritte Komponente, also als Coreaktant, wird in bekannter Weise zur Einstellung der gewünschten Oligomerisierungsgrade Glycerin zugesetzt.

Erfindungswesentlich ist nun die Abmischung der beschriebenen Polyesteroligomeren mit wasserlöslichen Polymeren.

Als wasserlösliches Polymer kommt Polyethylenglykol in Betracht. Dieses weist ein Molgewicht von 8 000 bis 10 000 auf. In einer besonders bevorzugten Ausführungsform dieser Erfindung wird PEG 8000 verwendet.

Die Abmischung mit den Polyesteroligomeren erfolgt vorzugsweise so, daß der Gehalt an wasserlöslichen Polymeren 5 - 50 % beträgt. Insbesondere bevorzugt beträgt der Gehalt 15 - 30 %.

In besonders bevorzugten Ausführungsformen der vorliegenden Erfindung sind die Polyesteroligomeren aufgebaut aus 1 Teil Glycerin und X Teilen Lactid und Y Teilen Glykolid, wobei X und Y jeweils unabhängig voneinander 1, 2 oder 3 bedeuten können.

Dabei sind insbesondere diejenigen besonders bevorzugt, bei welchen X + Y ≤ 4 ist.

Die Herstellung der Polyesteroligomeren erfolgt nach den bekannten und in den voranstehend zitierten Schriften beschriebenen üblichen Kondensationsverfahren und muss daher hier nicht näher beschrieben werden. So sind auch die Polyesteroligomere durch ein mittleres Molekulargewicht im Bereich von 200 bis 1 500 und vorzugsweise im Bereich von 300 bis 1 000 gekennzeichnet.

Besonders bevorzugt sind Ausführungsformen, in welchen die Polyesteroligomeren zusammengesetzt sind aus 1 Teil Glycerin, 2 Teilen Lactid und 1 Teil Glykolid oder auch aus 1 Teil Glycerin und jeweils 2 Teilen Lactid und Glykolid.

In einer ganz besonders bevorzugten Ausführungsform besteht das Polyesteroligomer aus 1 Teil Glycerin, 1 Teil Lactid und 3 Teilen Glykolid.

Femer wurde überraschenderweise festgestellt, daß sich auch bevorzugte Produkte herstellen lassen, wenn man während der Kondensationsreaktion eine Temperatur von 140° bis 160°C, vorzugsweise von 145° bis 155°C, wählt. Diese Temperaturen liegen im Vergleich zu den sonst bei Kondensationsreaktionen üblichen Temperaturen niedriger.

Die Oligomeren können nun entweder sterilisiert werden - vorzugsweise γ-Sterilisation - oder aber auch direkt weiter verarbeitet werden.

Die Abmischung solcher Polyesteroligomeren mit 15 - 30 % wasserlöslichem Polymer führt zu erfindungsgemäß besonders bevorzugten Knochensiegelmassen.

Die Abmischung der Polyesteroligomeren mit den wasserlöslichen Polymeren kann einmal durch Kneten per Hand erfolgen oder - was bevorzugt und einfacher zu handhaben ist - durch einen Kneter bei etwas erhöhten Temperaturen. Vorzugsweise wird zur vollständigen Entgasung noch Vakuum angelegt.

Die Produkte werden vorzugsweise sterilisiert gelagert, wobei bevorzugt eine γ-Sterilisation durchgeführt wird.

Die erfindungsgemäßen Knochensiegel werden zur Stillung lokaler Blutungen am Knochen verwendet. Im Hinblick auf ihre Anwendung zeigen sie viele verbesserte Eigenschaften.

Die erfindungsgemäßen Eigenschaften werden wie üblich an anästhesierten Schweinen nach Thorakotomie durch direktes Auftragen auf das Stemum getestet. Unter anderem sind die Adhäsionseigenschaften der Abmischung auf dem spongiösen Knochen des Sternums, die Beurteilung der Klebrigkeit am chirurgischen Handschuh, die Auftragseigenschaften, die Beurteilung der Blutstillung nach bestimmten Zeitspannen sowie die Beurteilung des Eindringungsvermögens in die Spongiosa und Beurteilung des Oberflächenfilms die Prüfkriterien.

Die verbesserten Knochenwachse zeigen nun eine geringe Adhäsion an Kunststoffe (z.B. chirurgische Handschuhe), sie weisen eine verbesserte Viskosität bei Raumtemperatur und Körpertemperatur auf, eine verbesserte Extrudierbarkeit und sie zeigen hervorragende blutstillende Eigenschaften.

Dies führt zu einer hervorragenden praktischen Handhabbarkeit dieser Knochensiegel, die die Arbeit des Operateurs wesentlich erleichtern. Die "Klebrigkeit" der Wachse ist wesentlich verringert, sowie auch das Fadenziehen, das bisher bei Produkten mit Milchsäure große Schwierigkeiten bereitet hatte. Femer ist auch die gute Gewebeverträglichkeit gewährleistet.

Mit den in dieser Erfindung beschriebenen Knochensiegeln stehen nun verbesserte Produkte zur Verfügung, denen eine beträchtliche Bedeutung zukommen.

Die folgenden Beispiele sollen die Erfindung näher erläutern.

### Beispiel A

### Allgemeine Vorschrift zur Herstellung der Polyesteroligomeren

Glycerin, Lactid und Glycolid werden in die Apparatur eingewogen und der Katalysator zupipettiert. Unter Stickstoff und Rühren wird der Kristallbrei innerhalb 1 h auf 100°C erwärmt, innerhalb 15 min. bei 100°C aufgeschmolzen, dann in 1 h auf 195°C erhitzt und 5 h bei 193-197°C gelassen. Danach wird das Ölbad entfernt und das Produkt heiß abgefüllt.

In Beispiel 1 wurde eine Zusammensetzung von Glycerin:L-Lactid:Glycolid von 1:2:1, in Beispiel 2 von Glycerin:D,L-Lactid:Glycolid von 1:1:3 gewählt.

In Tabelle 1 sind die Ansätze zusammengefasst.

**Tabelle 1**

| Beispiel | Glycerin | Lactid | Glycolid | Katalys * | Auswaage | Produkt |
|---|---|---|---|---|---|---|
| 1 | 1,6 M | L-; 3,2 M | 1,6 M | 2,24 ml | 787 g | klar, farbl. |
| 2 | 1,3 M | DL; 1,3 M | 3,9 M | 2,21 ml | 749 g | klar, farbl. |

| | | | | | | |
|---|---|---|---|---|---|---|
| *Katalysator: H₃PO₄ 0,5 % (bezogen auf Lactid- und Glycolidmenge) | | | | | | |

Die Proben wurden dann zur γ-Sterilisation in Cryo Vials abgefüllt.

### Beispiel B

### Alternative allgemeine Vorschrift zur Herstellung der Polyesteroligomeren

In die Apparatur werden Glycerin, Lactid (L-Lactid oder D,L-Lactid) und Glycolid eingewogen und der Katalysator zupipettiert. Unter Stickstoff und Rühren wird der Kristallbrei innerhalb 1 h auf 100°C erwärmt, innerhalb 15 min. bei 100°C aufgeschmolzen, dann in 30 min. auf 150°C erhitzt und man lässt dann 5 h bei 148-152°C reagieren. Danach wird das Ölbad entfernt und das Produkt heiß abgefüllt.

In Beispiel 3 wurde ein Verhältnis von Glycerin:D,L-Lactid:Glycolid von 1:1:3, in Beispiel 4 ein Verhältnis von Glycerin:D,L-Lactid:Glycolid von 1:2:2 gewählt.

In Tabelle 2 sind die Ansätze zusammengefasst.

**Tabelle 2**

| Beispiel | Glycenn | Lactid | Glycolid | Kataly.* | Produkt |
|---|---|---|---|---|---|
| 3 | 1,3 M | 1,3 M | 3,9 M | 2,21 ml | klar, farbl. |
| 4 | 1,3 M | 2,6 M | 2,6 M | 2,34 ml | Klar, farbl. |

| | | | | | |
|---|---|---|---|---|---|
| *Katalysator: H₃PO₄ 0,5 % (bezogen auf Lactid- und Glycolidmenge) | | | | | |

Die Proben wurden dann zur γ-Sterilisation in Cryo Vials abgefüllt.

### Beispiel C

### Allgemeine Vorschrift zur Abmischung von Polyesteroligomeren, hergestellt nach Beispielen A und B, mit Polyethylenglykol( PEG) im Kneter

Der Kneter wird zunächst mittels Wasserbad erwärmt, gleichzeitig wird im Wasserbad das entsprechende Copolymer miterhitzt (Wasserbad 90°C). Dann wird bei einer Knetertemperatur von ca. 70°C dem Knochensiegel portionsweise das PEG zugegeben und zwischendurch kurz untergeknetet. Das PEG schmilzt dann nach kurzer Zeit, danach vorsichtig minimales Vakuum angelegen (Aufschäumen). Die Mischung wirddann bei ca. 80°C 1,5 h geknetet. Man schaltet den Kneter ab und lässt bei vollem Vakuum noch ca. 30 min. stehen. Die Proben können dann abgefüllt und einer γ-Sterilisation unterworfen werden.

### Beispiel 5

Das Glycero-oligo-L-lactid-co-glycolid aus Beispiel 1 (Verhältnis 1:2:1) wird mit PEG 10 000 (Fluka, # 81268) nach der allgemeinen Vorschrift im Verhältnis 75 % zu 25 % PEG gemischt und anschließend sterilisiert. Man erhält einen Knochensiegel, der sich gut auftragen lässt.

### Beispiel 6

Das Glycero-oligo-L-lactid-co-glycolid aus Beispiel 1 (1:2:1) wird mit PEG 8000 (Fluka, # 81268) nach Vorschrift C im Verhältnis 60 % zu 40 % PEG gemischt und anschließend sterilisiert. Der fertige Knochensiegel zeigt eine sehr geringe Klebrigkeit am Handschuh, ist weich und lässt sich gut auftragen und zeigt eine gute Blutstillung.

### Beispiel 7

Der Polyoligoester (D,L-Lactid) aus Beispiel 2 (1:1:3) wird mit PEG 8000 (Fluka, #81268) nach Vorschrift C im Verhältnis 80 % zu 20 % PEG gemischt und anschließend γ-sterilisiert.

### Beispiel 8

Das Glycero-oligo-D,L-lactid-co-glycolid aus Beispiel 4 (1:2:2) wird mit PEG 8000 (Fluka, #81268) nach der allgemeinen Mischungsvorschrift im Verhältnis 70 % zu 30 % PEG gemischt. Man erhält einen hervorragend geeigneten Knochensiegel. Dieses Produkt klebt nicht, lässt sich sehr gut auftragen, die Blutstillung ist hervorragend.

### Beispiel 9

Das Glycero-oligo-D,L-lactid-co-glycolid aus Beispiel 3 (1:1:3) wird mit PEG 8000 (Fluka, #81268) nach der allgemeinen Vorschrift C im Verhältnis 70 % zu 30 % PEG gemischt und anschließend γ-sterilisiert. Dies führt zu einem hervorragend geeigneten Knochensiegel, welcher nicht klebrig ist, eine gute Konsistenz besitzt, sich gut auftragen lässt und einen gleichmäßigen Film bildet. Zudem ist die Blutstillung bei diesem Knochensiegel sehr gut.

## Patentansprüche

1. Knochensiegel, geeignet zum Stillen lokaler Blutungen am Knochen, bestehend aus Polyesteroligomeren der Milchsäure, Glykolsäure und Glycerin, **dadurch gekennzeichnet, dass** es zusätzlich aus Polyethylenglykol (PEG) mit einem Molgewicht des PEG von 8000 bis 10000, besteht.

2. Knochensiegel nach Anspruch 1, **dadurch gekennzeichnet, dass** die Polyesteroligomeren aufgebaut sind aus 1 Teil Glycerin und X Teile Lactid und Y Teile Glykolid, wobei X und Y jeweils unabhängig voneinander 1, 2 oder 3 bedeuten.

3. Knochensiegel nach Anspruch 2, **dadurch gekennzeichnet, dass** X + Y ≤ 4 ist.

4. Knochensiegel nach einem der Ansprüche 1 bis 3, **dadurch gekennzeichnet, dass** der Gehalt an wasserlöslichen Polymeren 5 - 50 % aufweist.

5. Knochensiegel nach Anspruch 4, **dadurch gekennzeichnet, dass** der Gehalt 15 - 30 % aufweist.

6. Knochensiegel nach Anspruch 1, **dadurch gekennzeichnet, dass** das Polyesteroligomere aufgebaut ist aus 1 Teil Glycerin, 1 Teil D, L-Lactid und 3 Teilen Glykolid und dass dieses mit dem wasserlöslichen Polymeren PEG 8000 in einem Verhältnis von 70% zu 30 % (PEG) abgemischt wird.

7. Polyesteroligomere, aufgebaut aus Glycerin, Lactid und Glykolid, zusammen mit Polyethylenglykol (PEG) mit einem Molgewicht von 8000 bis 10000 zur Verwendung zum Stillen lokaler Blutungen am Knochen.

## Claims

1. Bone sealant, suitable for staunching local haemorrhages on the bone, consisting of polyester oligomers of lactic acid, glycolic acid and glycerol, **characterized in that** it additionally consists of polyethylene glycol (PEG) having a molecular weight of the PEG of 8000 to 10,000.

2. Bone sealant according to Claim 1, **characterized in that** the polyester oligomers are synthesized from 1 part of glycerol and X parts of lactide and Y parts of glycolide, where X and Y in each case independently of one another are 1, 2 or 3.

3. Bone sealant according to Claim 2, **characterized in that** X + Y is ≤ 4.

4. Bone sealant according to one of Claims 1 to 3, **characterized in that** the content of water-soluble polymers is 5-50%.

5. Bone sealant according to Claim 4, **characterized in that** the content is 15-30%.

6. Bone sealant according to Claim 1, **characterized in that** the polyester oligomer is synthesized from 1 part of glycerol, 1 part of D,L-lactide and 3 parts of glycolide and **in that** this is blended with the water-soluble polymer PEG 8000 in a ratio of 70% to 30% (PEG).

7. Polyester oligomers synthesized from glycerol, lactide and glycolide, together with polyethylene glycol (PEG) having a molecular weight of 8000 to 10,000, for use in staunching local haemorrhages on the bone.

## Revendications

1. Cire osseuse appropriée pour arrêter les saignements locaux sur l'os, constituée de polyesters oligomères de l'acide lactique, de l'acide glycolique et de la glycérine, **caractérisée en ce qu'**elle comprend en plus du polyéthylèneglycol (PEG) ayant un poids moléculaire compris entre 8000 et 10 000.

2. Cire osseuse selon la revendication 1, **caractérisée en ce que** les polyesters oligomères sont formés à partir d'une partie de glycérine, de X parties de lactide et de Y parties de glycolide, X et Y étant égaux, indépendamment l'un de l'autre, à 1, 2 ou 3.

3. Cire osseuse selon la revendication 2, **caractérisée en ce que** X + Y est ≤ 4.

4. Cire osseuse selon l'une des revendications 1 à 3, **caractérisée en ce que** la teneur en polymères solubles dans l'eau est comprise entre 5 et 50 %.

5. Cire osseuse selon la revendication 4, **caractérisée en ce que** la teneur en polymères solubles dans l'eau est comprise entre 15 et 30 %.

6. Cire osseuse selon la revendication 1, **caractérisée en ce que** le polyester oligomère est formé à partir d'une partie de glycérine, d'une partie de D,L-lactide et de trois parties de glycolide et **en ce que** celui-ci est mélangé avec le polymère PEG 8 000 soluble dans l'eau dans un rapport compris entre 70 et 30 % (PEG).

7. Polyester oligomère formé à partir de glycérine, de lactide et de glycolide, associé à du polyéthylèneglycol (PEG) ayant un poids moléculaire compris entre 8 000 et 10 000, destiné à être utilisé pour arrêter les saignements locaux sur l'os.
